# EUROPEAN PATENT APPLICATION

(11) **EP 0 743 318 A1**
(43) Date of publication of application: **20.11.1996**
(21) Application number: 95938621.0
(22) Date of filing: 01.12.1995
(51) Int. Cl.: C07H 21/04, A61K 31/70

(54) **NOVEL ANTI-HIV AGENT**

(30) Priority: 02.12.1994 JP 332285/94; 06.12.1994 JP 333206/94; 06.12.1994 JP 333207/94; 06.12.1994 JP 333208/94; 06.12.1994 JP 333209/94
(71) Applicant: KAJI, Akira, Tokyo 203 (JP)
(72) Inventor: KAJI, Akira, Tokyo 203 (JP); KAJI, Hideko, Tokyo 203 (JP)
(74) Representative: Vossius, Volker, Dr.
(86) International application number: JP9502453
(87) International publication number: WO9616977

(57) **Abstract**

An anti-HIV agent which comprises as an active ingredient one or more selected from phosphorothioate bonding type or phosphodiester bonding type oligodeoxynucleotides having 2 to 8 bases of the below formulae (I), (II), (III), (IV) and (V):

(Gs)nG (I)

(Gs)nGp (II)

p(Gs)nG (III)

pGs(Gs)nGp (IV)

pGpGpGpGp (V)

wherein G represents deoxyguanosine structure, p is a phosphoric acid residue which forms an ester bonding with the adjacent G, s is the phosphorothiophosphoric acid residue which forms a phosphorothioester bonding with the adjacent G, and n is an integer of from 2 to 7.

## Description

### Field of the Industrial Application

The present invention relates to an anti-HIV (Human Immunodeficiency Virus) agent containing as an active ingredient a phosphorothioate bonding type or phosphodiester bonding type oligodeoxynucleotide which shows an excellent anti-viral action for diseases caused by HIV.

### Background Art

As a previous finding on oligohomodeoxynucleotides of DNA, Agrawal et al. reported that phosphorothioates with chain lengths of 15 bases and 20 bases were active for inhibition of HIV replication and that their effect depended on the chain length but not on a kind of the base constituting them (Proc. Natl. Acad. Sci. USA, 85, 7079, 1988).

Additionally, Matsukura et al. reported that a deoxycytidine phosphorothioate with 28 bases had an activity for inhibition of HIV replication (Proc. Natl. Acad. Sci. USA, 84, 7706, 1987). Moreover, Wyatt et al. recently reported that a phosphorothioate with 8 bases had an activity for inhibition of HIV replication (Proc. Natl. Acad. Sci. USA, 91, 1356, 1994).

On the other hand, as a previous finding on phosphodiester bonding type homooligomers, Agrawal et al. reported that homooligomers with 15 bases of 2'-deoxyadenosine (hereinafter abbreviated as A), 2'-deoxyguanosine (hereinafter abbreviated as G), 2'-deoxycytosine (hereinafter abbreviated as C) and 2'-deoxycytidine (hereinafter abbreviated as T) were active for inhibition of HIV replication. As for their effectiveness, they reported that the homooligomers, as having such a long chain length, might favorably act as a template of reverse transcriptase and be competitive to HIV nucleic acids (the above mentioned report).

Majumdar et al. reported that a phosphodiester type homooligomer with 28 bases of C was active for inhibition of HIV replication (Biochemistry, 28, 1340, 1989).

Crooke having generalized from these findings, reported that the activity of homopolymers of C was the highest, that the activity of homopolymers of T was higher in some degree than that of homopolymers of A and that the activity of these homopolymers depended on their chain lengths while exemplifying the activity of a homopolymer with 28 bases of C (Anti Cancer Drug Design, 6, 609, 1991). However, since all these nucleotides that have heretofore been described above to be active for inhibition of HIV replication have the chain length with 8 bases or more, they are problematic not only in their preparation but also in their absorbability and stability when they are administered into a body.

In order to solve these problems, the inventors made an extensive research on obtaining low-molecular oligonucleotides with an activity for inhibition of HIV replication, and recently found that short-chain oligodeoxyguanylic acids of between 4 and 8 bases (PCT/JP 95/00543) and short-chain oligoguanylic acids of between 2 and 8 bases (PCT/JP 95/01456) had the activity for inhibition of HIV replication.

As a result of further studies, the inventors newly found that a phosphorothioate bonding type and 3'-terminal and/or 5'-terminal phosphorylated phosphorothioate bonding type short-chain oligodeoxyguanylic acid had an excellent activity for inhibition of HIV replication not only in the initial infection but also in the chronic infection.

Furthermore, the inventors intensively studied short-chained, diester bonding type homooligomers of A, G, C and T with respect to their anti-HIV activity and, as a result, previously found that those of 15 bases of A, C and T were not active but in G a low molecular (short-chain) homooligomer of 4 bases had an anti-HIV activity and that the activity of the latter one was enhanced by its 5'-phosphorylation and 3'-phosphorylation. In addition, the inventors have found that oligomers phosphorylated at both terminals also have an enhanced anti-HIV activity. On the basis of these findings, the inventors have completed the invention.

Accordingly, the invention provides a novel anti-HIV agent characterized in that it comprises as an active ingredient one or more selected from phosphorothioate bonding type or 3'-terminal and/or 5'-terminal phosphorylated phosphorothioate bonding type oligodeoxynucleotides represented by the general formulae (I) to (V) described below in (a) to (e), and the 3'-terminal and 5'-terminal phosphorylated phosphodiester bonding type oligodeoxynucleotide represented by the general formula (V) described in (e).
(a) An anti-HIV agent which comprises as an active ingredient one or more selected from phosphorothioate bonding type oligodeoxynucleotides represented by the following general formula (I):

   (Gs)nG (I)

   wherein G represents deoxyguanosine structure, s is a phosphorothiophosphoric acid residue which forms an ester bonding with the adjacent G, each s forms the ester bondings with the 3'-hydroxyl of the left side G and with the 5'-hydroxyl of the right side G in the formula respectively, and n is an integer of from 2 to 7.
(b) An anti-HIV agent which comprises as an active ingredient one or more selected from 3'-terminal phosphorylated phosphorothioate bonding type oligodeoxynucleotides represented by the general formula (II):

   (Gs)nGp (II)

   wherein G represents deoxyguanosine structure, s is a phosphorothiophosphoric acid residue which forms an ester bonding with the adjacent G, each s forms the ester bondings with the 3'-hydroxyl of the left side G and with the 5'-hydroxyl of the right side G in the formula respectively, p at the right terminal is a phosphoric acid residue which forms an ester bonding with the adjacent G and p forms the ester bonding with the 3'-terminal hydroxyl group, and n is an integer of from 2 to 7.
(c) An anti-HIV agent which comprises as an active ingredient one or more selected from 5'-terminal phosphorylated phosphorothioate bonding type oligodeoxynucleotides represented by the general formula (III):

   p(Gs)nG (III)

   wherein G represents deoxyguanosine structure, s is a phosphorothiophosphoric acid residue which forms an ester bonding with the adjacent G, each s forms the ester bondings with the 3'-hydroxyl of the left side G and with the 5'-hydroxyl of the right side G in the formula respectively, p at the left terminal is a phosphoric acid residue which forms an ester bonding with the adjacent G, and p forms the monoester bonding with the 5'-terminal hydroxyl group, and n is an integer of from 2 to 7.
(d) An anti-HIV agent which comprises as an active ingredient one or more selected from 3'-terminal and 5'-terminal phosphorylated phosphorothioate bonding type oligodeoxynucleotides represented by the general formula (IV):

   pGs(Gs)nGp (IV)

   wherein G represents deoxyguanosine structure, s is a phosphorothiophosphoric acid residue which forms an ester bonding with the adjacent G, each s forms the ester bondings with the 3'-hydroxyl of the left side G and with the 5'-hydroxyl of the right side G in the formula respectively, p at the both terminals is a phosphoric acid residue which forms an ester bonding with the adjacent G, and forms the ester bonding with the 5'-hydroxyl (the left side) of the terminal G and the ester bonding with the 3'-hydroxyl (the right side) of the terminal G respectively, and n is an integer of from 1 to 5.
(e) An anti-HIV agent which comprises as an active ingredient a 3'-terminal and 5'-terminal phosphorylated phosphodiester bonding type oligodeoxynucleotide represented by the general formula (V):

   pGpGpGpGp (V)

   wherein G represents deoxyguanosine structure, p is a phosphoric acid residue which forms an ester bonding with the adjacent G, each terminal p forms the ester bonding with the 5'-hydroxyl (the left side) of the terminal G and with the 3'-hydroxyl (the right side) of the terminal G respectively, each in-between p of two Gs forms the ester bondings with the 3'-hydroxyl of the left side G and with the 5'-hydroxyl of the right side G in the formula respectively.

In the following, the invention is described in detail.

The oligodeoxynucleotides of the above general formulae (I) to (V) which are active ingredients of the anti-HIV agents of the invention can generally be synthesized by ordinary methods which are generally used in oligodeoxynucleotide synthesis (Scaringe et al., Nucleic Acids Res. 18, 5433; Damha and Ogilvie, Methods in Mol. Biol., 20, 81; Eckstein, Ann, Rev. Biochem., 54, 367). Some preferred, typical synthetic methods for the above oligodeoxynucleotides, which are the active ingredients of the anti-HIV agents of the invention, are mentioned below, as Synthetic Method A to Synthetic Method E.

### [Synthetic Method A]

In a case of the solid phase synthesis according to the phosphoramidite method, a starting material is prepared in which the 3'-terminal nucleoside of the aimed DNA is bonded to a support such as commercially available control pore glass (CPG). The 5'-hydroxyl protecting group, 4,4'-dimethoxytrityl group, of this nucleoside is removed, using trichloroacetic acid or the like. This is condensed with a commercially available amidite reagent (5'-o-dimethoxytrityl-3'-methoxy-N,N-diisopropylaminophosphine-deoxynucleotide) in which the amino group of the nucleic acid base as a second monomer from the 3'-terminal side is protected with isobutyryl group, using a condensing agent, tetrazole. In this case, the non-reacted 5'-hydroxyl group is preferably protected by acetylation or the like with acetic anhydride, dimethylaminopyridine or the like. This is followed by the sulfidation of the phosphorus part with tetraethylthiuram disulfide (or 3H-1,2-benzodithiol-3-one 1,1-dioxide) to lead to a phosphorothiotriester. This process is repeated until an oligonucleotide having the aimed chain length can be obtained. Finally, this is treated with thiophenol, by which each internucleotide of a triester form is converted into the corresponding diester form. This is followed by an ammonia treatment to detach from the support and to make the simultaneous deprotection of the base part, giving the aimed phosphorothioate bonding type oligodeoxynucleotide.

### [Synthetic Method B]

A starting material is used, in which the 3'-terminal phosphorylating reagent (2-[2-(4,4'-dimethoxytrityloxy) ethylsulfonyl]ethylsuccinic acid) is bonded to a support such as commercially available control pore glass (CPG). The protecting group, 4,4'-dimethoxytrityl group is removed, using trichioroacetic acid or the like. Next, this is condensed with a commercially-available monomer of an amidite reagent (5'-o-dimethoxytrityl-3'-methoxy-N,N-diisopropylaminophosphine-deoxyguanosine) in which the amino group of the nucleic acid base is protected with isobutyryl group, using a condensing agent, tetrazole. In this case, the non-reacted hydroxyl group is preferably protected by acetylation or the like with acetic anhydride, dimethylaminopyridine or the like. This is followed by oxidation of the phosphorous acid part with iodine to lead to a triester. Then, the 4,4'-dimethoxytrityl group the 5'-hydroxyl protecting group of the deoxynucleotide corresponding to the 3'-terminal is removed. Next, this is condensed with an amidite reagent which corresponds to the 2nd monomer from the 3'-terminal side, using a condensing agent, tetrazole. This is followed by the sulfidation of the phosphorus part with tetraethylthiuram disulfide (or 3H-1,2-benzothiol-3-one 1,1-dioxide) to lead to a phosphorothiotriester. After this, the above process is repeated until an oligonucleotide of, for example, formula (II) where n is from 3 to 7 can have the aimed chain length. Finally, this is treated with thiophenol, by which each internucleotide of a triester form is converted into the corresponding diester form. This is followed by an ammonia treatment for the detachment from the support and the simultaneous deprotection of the base part to give the aimed 3'-terminal phosphorylated phosphorothioate bonding type oligodeoxynucleotide.

### [Synthetic Method C]

A starting material is used, in which the 3'-terminal nucleoside of the aimed DNA has been bonded to a commercially available control pore glass (CPG) or the like support. The 5'-hydroxyl protecting group, 4,4'-dimethoxytrityl group, of this nucleoside is removed, using trichioroacetic acid or the like. This is condensed with a commercially available amidite reagent (5'-o-dimethoxytrityl-3'-methoxy-N,N-diisopropylaminophosphine-deoxynucleoside) in which the amino group of the nucleic acid base as a second monomer from the 3'-terminal side is protected with isobutyryl group, using a condensing agent, tetrazole. In this case, the non-reacted 5'-terminal hydroxyl group is preferably protected through acetylation or the like with acetic anhydride, dimethylaminopyridine or the like. This is followed by the sulfidation of the phosphorus part with tetraethylthiuram disulfide (or 3H-1,2-benzodithiol-3-one 1,1-dioxide) to lead to a phosphorothiotriester. This process is repeated until an oligonucleotide of, for example, formula (III) where n is from 3 to 8 can have the aimed chain length. Finally, this is condensed with a phosphorylating reagent (2-[2-(4,4'-dimethoxytrityloxy] ethylsulfonyl-(2-cyanoethyl)-(N,N-diisopropyl)-phosphor-amidite) at the 5'-terminal and is followed by the oxidation of the phosphorus part with iodine to lead to a triphosphate. Next, this is treated with thiophenol, by which each internucleotide of a triester form is converted into the corresponding diester form. This is followed by an ammonia treatment for the detachment from the support and the simultaneous deprotection of the base part to give the aimed 5'-terminal phosphorylated phosphorothioate bonding type oligodeoxynucleotide.

### [Synthetic Method D]

A starting material is used, in which a 3'-terminal phosphorylating reagent (2-[2-(4,4'-dimethoxytrityloxy) ethylsulfonyl]ethylsuccinic acid) is bonded to a commercially available control pore glass (CPG) or the like support. The protecting group, 4,4'-dimethoxytrityl group is removed, using trichloroacetic acid or the like. Next, this is condensed with a commercially available monomer of an amidite reagent (5'-dimethoxytrityl-3'-methoxy-N,N-diisopropylaminophosphine-deoxyguanosine) in which the amino group of the nucleic acid base has been protected with isobutyryl group, using a condensing agent, tetrazole. In this case, the non-reacted hydroxyl group is preferably protected through acetylation or the like with acetic anhydride, dimethylaminopyridine or the like. This is followed by the sulfidation of the phosphorus part with tetraethylthiuram disulfide (or 3H-1,2-benzodithiol-3-one 1,1-dioxide) to lead to a phosphorothiotriester. This process is repeated until an oligonucleotide having the aimed chain length can be obtained. Next, this is condensed with a phosphorylating reagent (2-[2-(4,4'-dimethoxytrityloxy]ethylsulfonyl-(2-cyanoethyl)-(N,N-diisopropyl)-phosphoramidite) at the 5'-terminal and is followed by the oxidation of the phosphorus part with iodine to lead to a triphosphate. Next, this is treated with thiophenol, by which each internucleotide of a triester form is converted into the corresponding diester form. This is followed by an ammonia treatment for the detachment from the support and the simultaneous deprotection of the base part to give the aimed 3'-terminal and 5'-terminal phosphorylated phosphorothioate bonding type oligodeoxynucleotide of the above general formula (IV).

### [Synthetic Method E]

A starting material is used, in which a 3'-terminal phosphorylating reagent (2-[2-(4,4'-dimethoxytrityloxy) ethylsulfonyl]ethylsuccinic acid) is bonded to a commercially available control pore glass (CPG) or the like support. The protecting group, 4,4'-dimethoxytrityl group is removed, using trichloroacetic acid or the like. Next, this is condensed with a commercially available monomer of an amidite reagent (5'-dimethoxytrityl-3'-methoxy-N,N-diisopropylaminophosphine-deoxyguanosine) in which the amino group of the nucleic acid base has been protected with isobutyryl group, using a condensing agent, tetrazole. In this case, the non-reacted hydroxyl group is preferably protected through acetylation or the like with acetic anhydride, dimethylaminopyridine or the like. This is followed by oxidation of the phosphorous acid part with iodine to lead to a triester. This process is repeated until an oligonucleotide having the intended chain length can be obtained. Next, this is condensed with a phosphorylating reagent (2-[2-(4,4'-dimethoxytrityloxy) ethylsulfonyl-(2-cyanoethyl)-(N,N-diisopropyl)-phosphoramidite]) at the 5'-terminal and is followed by the oxidation of the phosphorus part with iodine to lead to a triphosphate. Next, this is treated with thiophenol, by which each internucleotide of a triester form is converted into the corresponding diester form. This is followed by an ammonia treatment for the detachment from the support and the simultaneous deprotection of the base part to give the aimed 3'-terminal and 5'-terminal phosphorylated phosphodiester bonding type oligodeoxynucleotide of the above general formula (V).

The short-chain oligodeoxyguanylic acids which constitute the active ingredients in the anti-HIV agents of the invention can be commercially available products (for example, synthesized and purified by Oligo Etcetera Co., Wilson Bild., Oregon, USA).

Experimental examples are given below to explain the invention in more detail, but the invention is not limited thereby in any way.

### Experimental Example 1

### 〈Anti-HIV activity tests using MT-4 cells (T cell line)〉

Onto a 96-well microplate which contained each oligodeoxynucleotide as previously diluted stepwise were placed MT-4 cells (6 x 10⁴ cells per well). The cells were then infected with AIDS virus [HIV-1(IIIB) OR HIV-2(ROD) : ca. 50 TCID₅₀ (50 % tissue culture infectious doses)/well] and were incubated in a 5 % CO₂ atmosphere at 37°C for 5 days. For evaluation of the inhibition of the cytopathic effect by the infection, viable cells were counted by an MTT method (see J. Virol. Methods, 20, 309, 1988) after the 5-day incubation. The results are shown in Table 1 and Table 2. In the formula of each oligonucleotide in these tables, A is deoxyadenosine, C is deoxycytidine, T is thymidine, and G, p and s have the same meaning as mentioned above.

**Table 1**

| | ED₅₀ to HIV-1 (effective dose for 50% cytopathic inhibitory concentration) | CD₅₀ | SI |
|---|---|---|---|
| GsG | 100 µg/ml or more | | |
| GsGsG | 35.5 µg/ml | | |
| GsGsGsG | 8.5 µg/ml | | |
| GsGsGsGsG | 2.6 µg/ml | | |
| AsAsAsAsAsA | 100 µg/ml or more | | |
| CsCsCsCsCsC | 100 µg/ml or more | | |
| TsTsTsTsTsT | 100 µg/ml or more | | |
| GsGp | 100 µg/ml or more | | |
| GsGsGp | 12.7 µg/ml | | |
| GsGsGsGp | 4.9 µg/ml | | |
| pGsGsG | 45.4 µg/ml | | |
| pGsGsGsG | 3.4 µg/ml | | |
| pGsGsGsGsGsGsGsG | 1.4 µg/ml | | |
| AsAsAsAsAsA | 100 µg/ml or more | | |
| CsCsCsCsCsC | 100 µg/ml or more | | |
| TsTsTsTsTsT | 100 µg/ml or more | | |
| pGsGsGp | 52.3 µg/ml | | |
| pGsGsGsGp | 10.3 µg/ml | | |
| GpGpGp | 100 µg/ml or more | 100 µg/ml or more | |
| pGpGpGpGp | 40.0 µg/ml | 100 µg/ml or more | 2.5 or more |

As in Table 1, the phosphorothioate bonding type or phosphodiester bonding type oligoguanylic acids of the above general formulae (I) to (V), which are the active ingredients in the anti-HIV agents of the invention, showed an extremely excellent anti-HIV activity against HIV-1.

On the other hand, short-chained phosphorothioate bonding type oligodeoxyadenylic acid, deoxycytidylic acid, and thymidylic acid oligodeoxynucleotide composed of 6 bases were not effective against HIV-1. From the data in Table 1, it is demonstrated that the usefulness of the phosphorothioate bonding type or phosphodiester bonding type oligodeoxyguanylic acids of the invention is remarkable.

**Table 2**

| | ED₅₀ to HIV-2 (effective dose for 50% cytopathic inhibitory concentration) |
|---|---|
| pGsGsGsG | 7.1 µg/ml |

As in Table 2, the 5'-terminal phosphorylated phosphorothioate bonding type oligodeoxyguanylic acid of the present invention, which is composed of 4 bases, also showed an excellent anti-HIV effect against HIV-2.

The compounds shown in Table 1 and Table 2 had no cytotoxicity at the concentration of 100 µg/ml.

### Experimental Example 2

### 〈Anti-HIV activity tests using chronically HIV-1-infected cells〉

Phosphorothioate bonding type oligodeoxyguanylic acids composed of 4 bases, which are the active ingredients in the anti-HIV agents of the invention, were tested on their activity against acute infection and chronic infection with HIV-1.

Onto a 96-well microplate were placed H9/IIIB cells (5 x 10⁴ cells per well) (see Science, 224, 497, 1984), which was chronically infected with HIV-1 and which all the time released HIV-1 in the culture. To this was added the phosphorothioate bonding type oligodeoxynucleotide, and the cells were incubated in a 5 % CO₂ atmosphere at 37°C for 5 days. The oligonucleotide was added only once on the first day or three times at 2 days intervals. The amount of HIV-1 released in the culture was measured, using a p24 antigen assay kit (produced by Coulter Co.). In order to check the cytotoxicity of the oligonucleotides used herein, the number of the viable cells in the culture was counted according to an MTT method. The results are shown in Table 3.

**Table 3**

| | Dose (µg/ml) | Number of Dosing Times | Amount of p24 (%, based on the control group) | Number of viable Cells |
|---|---|---|---|---|
| GsGsGsG | 100 | x 1 | 75 | |
| | 25 | x 3 | 89 | |
| | 50 | x 3 | 59 | |
| pGsGsGsG | 100 | x 1 | 34 | 85 |
| | 25 | x 3 | 61 | 95 |
| | 50 | x 3 | 24 | 82 |
| TsTsGsGsGsGsTsT | 100 | x 1 | 97 | 76 |
| | 50 | x 3 | 110 | 78 |

As in Table 3, the phosphorothioate bonding type and 5'-terminal phosphorylated phosphorothioate bonding type oligodeoxyguanylic acids composed of 4 bases, which are the active ingredients in the anti-HIV agents of the invention, were found effective against chronic infection with HIV-1, at the concentration at which the compounds had no cytotoxicity. The results obtained herein demonstrate that the compounds tested can be used as medicaments for AIDS therapy. As in Table 3 and as so reported by Wyatt et at., the phosphorothioate bonding type oligothymidylic acid-deoxyguanylic acid composed of 8 bases (TsTsGsGsGsGsTsT) was not effective against HIV-1 even though it was administered three times at the dose of 100 µg/ml, and the compound was ineffective against chronic infection with HIV-1. Thus, the results obtained herein also demonstrate that the compounds of the invention composed of 4 bases are much more effective than the compounds previously reported by Wyatt et al.

### Experimental Example 3

### 〈Anti-HIV activity tests using various drugs as administered at varying time schedule after infection with HIV-1〉

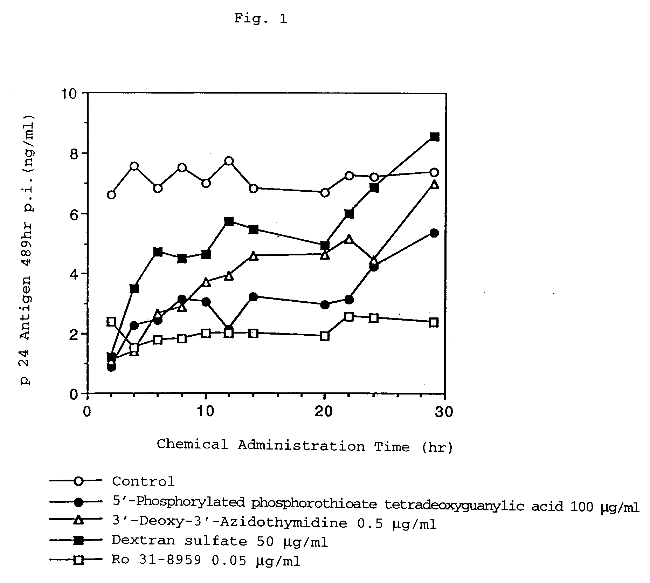

A 5'-terminal phosphorylated phosphorothioate bonding type oligodeoxyguanylic acid composed of 4 bases was tested whatever the stage whether the compound acts on in the propagation life cycle of infectious HIV-1 to show the anti-HIV activity of the compound against HIV-1. H9 cells were infected with HIV-1 and placed onto a 96-well microplate in an amount of 5 x 10⁴ cells per well. 100 µg/ml of the phosphorothioate bonding type oligodeoxyguanylic acid to be tested was added to the plate at varying time schedule, and the cells were incubated in a 5 % CO₂ atmosphere at 37°C for 48 hours. The amount of HIV-1 as released in the culture was measured, using a p24 antigen assay kit. As controls, 3'-deoxy-3'-azidothymidine, dextran sulfate (molecular weight: 8,000) and an HIV-1 protease inhibitor Ro 31-8959, of which the active sites are known, were used. The test results are shown Fig. 1 attached below.

From Fig. 1, the following facts are known. Dextran sulfate was effective just after the infection, but its effect was lowered when it was added in 4 hours and later after the infection. The effect of 3'-deoxy-3'-azidothymidine was gradually lowered with the lapse of time after the infection, and in 14 hours and later after the infection, its effect against the primary infection was lost like the effect of dextran sulfate. The HIV-1 protease inhibitor Ro 31-8959 was sufficiently effective, when added within 20 hours after the infection. As opposed to these controls, the 5'-terminal phosphorylated, phosphorothioate bonding type oligodeoxyguanylic acid composed of 4 bases of the invention showed the highest effect just after the infection, and its effect was, though being somewhat lowered with the lapse of time after the infection, still satisfactory even in 20 hours and later after the infection. Thus, the results obtained herein suggest that the phosphorothioate bonding type oligodeoxyguanylic acids which are the active ingredients in the anti-HIV agents of the invention, especially those of 4 bases, act on at least two stages in the cycle of HIV-1 infection (in the initial stage of HIV-1 infection and in the latter stage thereof where the viral gene was integrated with the host cell gene). Accordingly, it is expected that the active ingredients in the anti-HIV agents of the invention are active on plural stages in the cycle of HIV infection and that they will not induce drug-resistant viruses which are often problematic in a therapy.

As has been described in detail hereinabove, the oligodeoxyguanylic acids of the general formulae (I) to (V) which are the active ingredients in the anti-HIV agents of the invention exhibit an extremely excellent anti-HIV activity. In addition, the safety of the 5'-terminal phosphorylated phosphorothioate bonding type oligodeoxyguanylic acid composed of 4 bases was ascertained by the intravenous injection of the compound of 20 mg/kg in mice. In other experiments, it was also confirmed that the oral administration of the compound of 100 mg/kg resulted in no death of the tested animals. Thus, the high safety of the compounds of the invention was confirmed.

The novel anti-HIV agents of the invention are, in particular, useful in medicaments for treating diseases infected with retroviruses such as AIDS, and can be administered with no particular limitation in dosage forms. The compounds can be directly administered or can be combined with known pharmaceutical carriers into appropriate pharmaceutical preparations. As examples, illustrated are oral preparations such as tablets, capsules, granules, fine granules and powders, as well as parenteral preparations such as injections, drip infusions and suppositories.

The required dose for oral administration depends on the age and the body weight of each patient, and the severity of the disease. However, the daily dose to adults is preferably from 0.1 g to 6 g, which may be divided into plural portions for administration in plural times.

The oral preparations such as tablets, capsules and granules mentioned above can be prepared according to conventional methods using, for example, vehicles such as starch, lactose, sugar, mannitol, carboxymethyl cellulose and inorganic salts.

The preparations of this type may expediently contain any other additives such as binders, disintegrators, surfactants, lubricants, fluidity promoters, sweeteners, colorants and flavors, in addition to the above mentioned vehicles. Examples of the additives are mentioned below.

### [Binders]

Starch, dextrin, Arabic gum powder, gelatin, hydroxypropyl starch, methyl cellulose, sodium carboxymethyl cellulose, hydroxypropyl cellulose, crystalline cellulose, ethyl cellulose, polyvinyl cellulose, Macrogol.

### [Disintegrators]

Starch, hydroxypropyl starch, sodium carboxymethyl cellulose, calcium carboxymethyl cellulose, carboxymethyl cellulose, low-substituted hydroxypropyl cellulose.

### [Surfactants]

Sodium laurylsulfate, soya lecithin, sucrose fatty acid ester, Polysorbate 80.

### [Lubricants]

Talc, wax, hydrogenated vegetable oils, sucrose fatty acid ester, magnesium stearate, polyethylene glycol.

### [Flowing promoters]

Light silicic anhydride, dry aluminium hydride gel, synthetic aluminium silicate, magnesium silicate.

The oligodeoxynucleotides of the above general formulae (I) to (V) can be administered in any form of suspensions, emulsions, syrups and elixirs comprising them, which may optionally contain flavorings and colorants.

The required dose for a parenteral administration depends on the age and the body weight of the patient, and the severity of the disease. However, the daily dose to adults is preferably from about 1 mg to 100 mg a day, which may be administered through intravenous injection, intravenous drip infusion, subcutaneous injection or intramuscular injection.

The parenteral preparations can be prepared according to conventional methods, in which generally used is a diluent that may be selected from, for example, distilled water for injection, physiological saline, aqueous glucose solution, vegetable oils for injection, sesame oil, peanut oil, soya oil, corn oil, propylene glycol and polyethylene glycol. If desired, the preparations may contain microbicides, preservatives and stabilizers. From the viewpoint of their stability, the parenteral preparations are filled in vials or the like, then frozen and dried according to ordinary lyophilizing technology. The lyophilizate can be re-prepared into liquid preparations before use. Any of isotonic agents, stabilizers, preservatives and soothing agents and other additives can be expediently added to the preparations as required.

As other examples of the parenteral preparations, illustrated are embrocations such as externally applicable liquids and ointments, and suppositories for rectal administration. These can be prepared according to conventional methods.

Examples are given below to explain the invention in more detail, but the invention is not limited thereby in any way.

### Example 1

| Formulation: | |
|---|---|
| ① Crystalline cellulose | 84.5 wt.pts. |
| ② Magnesium stearate | 0.5 wt.pts. |
| ③ Calcium carboxymethyl cellulose | 5 wt.pts. |
| ④ pGsGsGsGsG | 10 wt.pts. |
| Total | 100 wt.pts. |

According to the above-mentioned formulation, ①, ④ and a part of ② were homogenized, then shaped under pressure, and powdered. The resulting powder was mixed with the remaining ② and ③, and shaped under pressure into tablets (200 mg/tablet), using a tabletting machine.

One tablet contained 20 mg of pGsGsGsGsG. From 4 to 8 tablets/day/adult are divided into plural portions, which are administered separately in a day.

Other tablets (200 mg/tablet) containing any of GsGsGsGsG, GsGsGsGp, pGpGpGpGp and pGsGsGsGp were prepared in accordance with the above-mentioned formulation of Example 1.

### Example 2

| Formulation: | |
|---|---|
| ① Crystalline cellulose | 35.4 wt.pts. |
| ② Ethanolic solution of 10 % hydroxypropyl cellulose | 50 wt.pts. |
| ③ Calcium carboxymethyl cellulose | 5 wt.pts. |
| ④ Magnesium stearate | 0.5 wt.pts. |
| ⑤ pGsGsGsG | 10 wt.pts. |
| Total | 100 wt.pts. |

According to the above-mentioned formulation, ①, ② and ⑤ were homogenized and then kneaded by an conventional method. The resulting mixture was dried and granulated, using an extrusion granulator, and then powdered. This was mixed with ③ and ④ and shaped under pressure into tablets (200 mg/tablet), using a tabletting machine.

One tablet contained 20 mg of pGsGsGsG. From 4 to 8 tablets/day/adult are divided into plural portions, which are administered separately in a day.

Other tablets (200 mg/tablet) containing any of GsGsGsG, GsGsGsGp, pGpGpGpGp and pGsGsGsGp were prepared in accordance with the above-mentioned formulation of Example 2.

### Example 3

| Formulation: | |
|---|---|
| ① Distilled water for injection | ad lib. |
| ② Glucose | 200 mg |
| ③ pGsGsGsG | 10 mg |
| Total | 15 ml |

② and ③ were dissolved in ①, and then the solution was filled into 5 ml ampoules and sterilized at 121°C for 15 minutes to obtain injections.

Other injections containing any of GsGsGsG, GsGsGsGp, pGsGsGsGp and pGpGpGpGp were prepared in accordance with the above-mentioned formulation of Example 3.

### Advantages of the Invention

The novel anti-HIV agents of the invention are effective for the treatment of diseases caused by HIV (AIDS virus) such as AIDS and ARC.

### Brief Description of the Drawing

Fig 1 shows the results of the anti-HIV activity tests where various chemicals were administered at varying time schedule after infection with HIV-1.

## Claims

1. An anti-HIV agent which comprises as an active ingredient one or more selected from phosphorothioate bonding type oligodeoxynucleotides represented by the general formula (I):
(Gs)nG (I)
wherein G represents deoxyguanosine structure, s is a phosphorothiophosphoric acid residue which forms an ester bonding with the adjacent G, each s forms the ester bondings with the 3'-hydroxyl of the left side G and with the 5'-hydroxyl of the right side G in the formula respectively, and n is an integer of from 2 to 7.

2. An anti-HIV agent which comprises as an active ingredient one or more selected from 3'-terminal phosphorylated phosphorothioate bonding type oligodeoxynucleotides represented by the general formula (II):
(Gs)nGp (II)
wherein G represents deoxyguanosine structure, s is a phosphorothiophosphoric acid residue which forms an ester bonding with the adjacent G, each s forms the ester bondings with the 3'-hydroxyl of the left side G and with the 5'-hydroxyl of the right side G in the formula respectively, p at the right terminal is a phosphoric acid residue which forms an ester bonding with the adjacent G, and p forms the ester bonding with the 3'-terminal hydroxyl group, and n is an integer of from 2 to 7.

3. The anti-HIV agent according to claim 2, which comprises as the active ingredient the 3'-terminal phosphorylated phosphorothioate bonding type oligodeoxynucleotide of the general formula (II) where n is 3, GsGsGsGp.

4. An anti-HIV agent which comprises as an active ingredient one or more selected from 5'-terminal phosphorylated phosphorothioate bonding type oligodeoxynucleotides represented by the general formula (III):
p(Gs)nG (III)
wherein G represents deoxyguanosine structure, s is a phosphorothiophosphoric acid residue which forms an ester bonding with the adjacent G, each s forms the ester bondings with the 3'-hydroxyl of the left side G and with the 5'-hydroxyl of the right side G in the formula respectively, p at the left terminal is a phosphoric acid residue which forms an ester bonding with the adjacent G, and forms the monoester bonding with the 5'-terminal hydroxyl group, and n is an integer of from 2 to 7.

5. The anti-HIV agent according to claim 4, which comprises as the active ingredient the 5'-terminal phosphorylated phosphorothioate bonding type oligodeoxynucleotide of the general formula (III) where n is 3.

6. An anti-HIV agent which comprises as an active ingredient one or more selected from 3'-terminal and 5'-terminal phosphorylated phosphorothioate bonding type oligodeoxynucleotides represented by the general formula (IV):
pGs(Gs)nGp (IV)
wherein G represents deoxyguanosine structure, s is a phosphorothiophosphoric acid residue which forms an ester bonding with the adjacent G, each s forms the ester bondings with the 3'-hydroxyl of the left side G and with the 5'-hydroxyl of the right side G in the formula respectively, p at the both terminals is a phosphoric acid residue which forms an ester bonding with the adjacent G, and forms the ester bonding with the 5'-hydroxyl (the left side) of the terminal G the ester bonding with the 3'-hydroxyl (the right side) of the terminal G respectively, and n is an integer of from 1 to 5.

7. The anti-HIV agent according to claim 6, which comprises as the active ingredient the 3'-terminal and 5'-terminal phosphorylated, phosphorothioate bonding type oligodeoxynucleotide of the general formula (IV) where n is 2.

8. An anti-HIV agent which comprises as an active ingredient a 3'-terminal and 5'-terminal phosphorylated phosphodiester bonding type oligodeoxynucleotide represented by the general formula (V):
pGpGpGpGp (V)
wherein G represents deoxyguanosine structure, p is a phosphoric acid residue which forms an ester bonding with the adjacent G, each terminal p forms the ester bonding with the 5'-hydroxyl (the left side) of the terminal G and with the 3'-hydroxyl (the right side) of the terminal G respectively, each in-between p of two Gs forms the ester bondings with the 3'-hydroxyl of the left side G and with the 5'-hydroxyl of the right side G in the formula respectively.
